# EUROPÄISCHE PATENTSCHRIFT

(11) **EP 1 485 352 B1**
(45) Veröffentlichungstag und Bekanntmachung des Hinweises auf die Patenterteilung: **29.06.2005**
(21) Anmeldenummer: 03743342.2
(22) Anmeldetag: 27.02.2003
(51) Int. Cl.: C07D 209/54, A61K 31/40

(54) **AZA-SPIROVERBINDUNGEN ZUR BEHANDLUG VON SCHMERZEN**
AZASPIRO COMPOUNDS FOR THE TREATMENT OF PAIN
COMPOSES AZASPIRO SERVANT A TRAITER LES DOULEURS

(30) Priorität: 07.03.2002 DE 10210190
(43) Veröffentlichungstag der Anmeldung: 15.12.2004
(60) Teilanmeldung: 04029311.0
(73) Patentinhaber: SCHWARZ PHARMA AG, D-40789 Monheim/Rhld. (DE)
(72) Erfinder: MEESE, Claus, 40789 Monheim (DE); SELVE, Norma, 53842 Troisdorf (DE); SCHMIDT, Dirk, 40597 Düsseldorf (DE)
(86) Internationale Anmeldenummer: PCT/EP2003/001986
(87) Internationale Veröffentlichungsnummer: WO 2003/074486

(56) Entgegenhaltungen:
- WO-A-99/61424
- GB-A- 967 718
- BADGER A M ET AL: "ANTIARTHRITIC AND SUPPRESSOR CELL INDUCING ACTIVITY OF AZASPIRANES:STRUCTURE-FUNCTION RELATIONSHIPS OF A NOVEL CLASS OF IMMUNOMODULATORY AGENTS" JOURNAL OF MEDICINAL CHEMISTRY, AMERICAN CHEMICAL SOCIETY. WASHINGTON, US, Bd. 33, Nr. 11, 1. November 1990 (1990-11-01), Seiten 2963-2970, XP000605424 ISSN: 0022-2623
- LUHNDAHL K ET AL: "SYNTHESIS AND ANTIVIRAL ACTIVITIES OF ADAMANTANE SPIRO COMPOUNDS. 1. ADAMANTANE AND ANALOGOUS SPIRO-3'-PYRROLIDINES" JOURNAL OF MEDICINAL CHEMISTRY,AMERICAN CHEMICAL SOCIETY,WASHINGTON,US, Bd. 15, Nr. 2, 1. Mai 1971 (1971-05-01), Seiten 129-131, XP002248546
- GROGAN C H ETAL: "SPIRANES.IV.ALKYL,CYCLOALKYL,ALKENYL,ARYL ,ARYLALKYL,AND HYDRAZONO AZASPIRANE DERIVATIVES" JOURNAL OF MEDICINAL CHEMISTRY,AMERICAN CHEMICAL SOCIETY.WASHINGTON,US, Bd. 7, 2. August 1963 (1963-08-02), Seiten 78-87, XP002248547
- DATABASE CA [Online] CHEMICAL ABSTRACTS SERVICE, COLUMBUS, OHIO, US; PANOUSE, J. J. ET AL: "Immunomodulator structure-activity relationships: contribution of molecular modeling" retrieved from STN Database accession no. 134:110100 XP002248548 & ANNALES PHARMACEUTIQUES FRANCAISES (2000), 58(5), 291-302 ,

## Beschreibung

Neuropathische Schmerzen sind eine schwierig zu behandelnde Form von chronischen Schmerzen, die durch Verletzungen oder Erkrankungen des peripheren und/oder zentralen Nervensystems hervorgerufen werden und die nur schlecht auf traditionelle Analgetika ansprechen.

Auf Grund der Ähnlichkeiten in der Pathophysiologie von Epilepsie und neuropathischem Schmerz werden in jüngster Zeit zunehmend antikonvulsive Wirkstoffe zur Behandlung von neuropathischem Schmerz herangezogen. Ein Beispiel hierfür ist Gabapentin, das als Antiepileptikum bereits längere Zeit zugelassen ist, in jüngster Zeit aber auch zunehmend Bedeutung in der Behandlung von neuropathischem Schmerz bekommt (Tremont-Lukats, Drugs 60, 2000, 1029; Bock, Nervenarzt 72, 2001, 69).

Obwohl der Wirkmechanismus von Gabapentin noch nicht völlig aufgeklärt ist, hat Gabapentin durch seine Beeinflussung der glutaminergen/GABAergen Übertragung und der Beeinflussung von Calciumkanälen ein breites Wirkspektrum, dass von der Behandlung der Epilepsie über neuropathische und andere Schmerzzustände, wie z.B. Migräneschmerz (Block, Nervenarzt 72, 2001, 69) oder Muskel- und Skelettschmerz (EP 1 047 414), bis hin zur Behandlung von Depressionen (EP 552240), neurodegenerativen Erkrankungen (EP 446570), Angst- und Panikzuständen (EP 804 182) oder Manien (EP 825 857) reicht.

Ein Nachteil von Gabapentin ist die Bildung von toxischem Gabapentin-Lactam (2-Azaspiro [4.5]decan-3-one) bei Lagerung, und die damit verbundene Schwierigkeit, stabile Gabapentin-Formulierungen herzustellen.

In der WO 99/25683 werden eine große Zahl von in Position 4 substituierten Pyrrolidinon-Verbindungen, die auch Azaspiroverbindungen wie Gabapentin-Lactam umfassen, zur Behandlung von Erkrankungen vorgeschlagen, die mit erhöhten Glutamatspiegeln einhergehen, wie z.B. Epilepsie, Alzheimer, ALS oder Parkinson. In einem in-vitro Modell wird die Wirksamkeit des Gabapentin-Lactams in lschämien und die Reduktion des Glutamat-Spiegels gezeigt. Ferner wird die neuroprotektive Wirkung von Gabapentin-Lactam in einem Ratten-Modell demonstriert. Auf Grund der Toxizität von Gabapentin-Lactam ist dies jedoch nicht zur Therapie am Menschen geeignet. Zudem gibt es in der WO 99/25683 keinen Hinweis darauf, dass die beanspruchten Pyrrolidone zur Behandlung von neuropathischem Schmerz geeignet sind.

In der DE 25 57 220 werden N-substituierte Gabapentin-Lactam-Derivate zur Behandlung von Epilepsie und zerebralen Störungen beschrieben. Die Verwendung bei Schmerzen wird nicht gelehrt.

Azaspiroverbindungen mit Aryl-Substituenten zur Behandlung von Schmerzen werden beschrieben in EP 337 547, EP 687 268, EP 880 528, EP 894 497, EP 906 315, EP 912 579, EP 929 554, EP 977 758 und EP 989 987. Es findet sich in diesen Dokumenten kein Hinweis darauf, dass auch Desaryl-Azaspiroverbindungen analgetisches Potential haben.

EP A 116 347 schlägt Amino-substituierte 1-Azaspiro[4.5.]decane und -undecane zur Behandlung von Schmerzen vor. 2-Azaspiroverbindungen werden nicht offenbart.

EP 310 321 beschreibt 2-Azaspiroverbindungen, in denen das Azäatom mit einer Stickstoff-haltigen Seitenkette substituiert ist. Die offenbarten Verbindungen werden als immunomodulatorisch beschrieben. Eine analgetische Wirkung wird nicht offenbart.

WO 99161424 beschreibt Aza-spiro-carbonsäuren, die u.a. analgetisches Potential haben

In der klinischen Praxis haben sich nur wenige Wirkstoffe bei der Behandlung von chronischen oder neuropathischen Schmerzen als wirksam und geeignet erwiesen, so dass in dieser Indikation weiterhin ein grosser Bedarf an innovativen Arzneimitteln besteht.

Ziel der vorliegenden Erfindung war es daher, alternative Arzneimittel zur Behandlung von Schmerz, insbesondere von chronischem, chronisch-entzündlichem und/oder neuropathischem Schmerz zur Verfügung zu stellen.

Dabei wurde überraschenderweise gefunden, dass von Gabapentin-Lactam abgeleitete Azaspiroverbindungen der Verbindungen wie beansprucht eine stärkere analgetische Potenz als Gabapentin und Gabapentin-Lactam aufweisen und zugleich eine geringere Toxizität als Gabapentin-Lactam zeigen.

Die erfindungsgemäßen Azaspiroverbindungen, die zur therapeutischen Verwendung geeignet sind, lauten wie folgt:
2-Azaspiro[4,5]decan-3-thion
2-Azaspiro[4,4]nonan-3-thion
2-Azaspiro[4,6]undecan-3-thion
N-(2-Azaspiro[4.5]decan-3)-oxim
N-(2-Azaspiro[4.6]undecan)-3-oxim
1-(2-Azaspiro[4.5]dec-2yl)-ethanon.
2-Azaspiro[4,5]decan

Ferner ist dem Fachmann klar, dass in Abhängigkeit von den Substituenten Azaspiroverbindungen in optisch inaktiver oder aktiver Form existieren können. Daher werden reine Enantiomere ebenso wie Razemate oder optisch inaktive Verbindungen explizit zum Gegenstand der Erfindung gemacht.

Ein weiterer Gegenstand der Erfindung sind pharmazeutische Zusammensetzungen, Azaspiroverbindungen I, wie oben beschrieben, sowie mindestens einen pharmazeutisch annehmbaren Hilfsstoff umfassen.

Dem Fachmann ist klar, dass die pharmazeutische Formulierung in Abhängigkeit vom beabsichtigten Applikationsweg unterschiedlich ausgestaltet sein kann. So kann die pharmazeutische Formulierung beispielsweise zur intravenösen, intramuskulären, intrakutanen, subkutanen, oralen, bukkalen, sublingualen, nasalen, transdermalen, inhaiativen, rektalen oder intraperitonealen Verabreichung angepasst sein.

Entsprechende Formulierungen und hierfür geeignete pharmazeutische Träger bzw. Hilfsstoffe wie Füllstoffe, Sprengmittel, Bindemittel, Gleitmittel, Stabilisatoren, Aromastoffe, Antioxidanzien, Konservierungsmittel, Dispersions- oder Lösungsmittel, Puffer, Elektrolyte, sind dem Fachmann auf dem Gebiet der Pharmazeutik bekannt und sind beispielsweise in Standardwerken wie Sucker, Fuchs und Speiser ("Pharmazeutische Technologie", Georg Thieme Verlag, Stuttgart) beschrieben.

In einer bevorzugten Ausführungsform der Erfindung werden die die erfindungsgemäßen Verbindungen enthaltenden pharmazeutischen Zusammensetzungen oral verabreicht und können beispielsweise als Kapsel, Tablette, Pulver, Granulat, Dragee oder in flüssiger Form vorliegen.

Alternative pharmazeutische Zubereitungen können beispielsweise Infusions- oder Injektionslösungen, Öle, Suppositorien, Aerosole, Sprays, Mikrokapseln oder Mikropartikel sein.

Dabei kann die Formulierung als schnell freisetzende Darreichungsform ausgestaltet sein, wenn ein rascher Wirkeintritt gewünscht ist, z.B. bei akutem, bzw. akut durchbrechendem chronischen bzw. neuropathischen Schmerz. Entsprechende orale Formulierungen sind beispielsweise beschrieben in EP 159 237 oder EP 1 126 821.

Ist dagegen eine protrahierte Freisetzung erwünscht, bietet sich eine Formulierung mit retardierter Wirkstofffreisetzung an. Entsprechende orale Formulierungen sind ebenfalls aus dem Stand der Technik bekannt.

Ein weiterer Gegenstand der Erfindung sind Verkaufspackungen, umfassend mindestens eine pharmazeutische Formulierung, wie oben beschrieben, sowie eine Anleitung zu deren Verwendung. Eine solche Verkaufspackung kann auch weitere Arzneimittel enthalten. Zum Beispiel könnte die Verkaufspackung zusätzlich ein weiteres Analgetikum, ein Sedativum, ein Ergotamin-Derivat, ein Antiemetikum, ein Antiphlogistikum oder ein Antidepressivum enthalten.

Überraschenderweise zeigen die erfindungsgemäßen Verbindungen in pharmakologischen Vergleichstests eine starke Wirksamkeit im Formalintest, einem in-vivo Test für die Voraussage der potentiellen Wirksamkeit einer Substanz bei der Behandlung von chronischem, bzw. chronisch-entzündlichem und/oder neuropathischem Schmerz (Tjolsen and Herle, Handbook Exp. Pharmacol. Vol 130, Ed: Dickensen & Besson, Springer Verlag 1997, Seite 6).

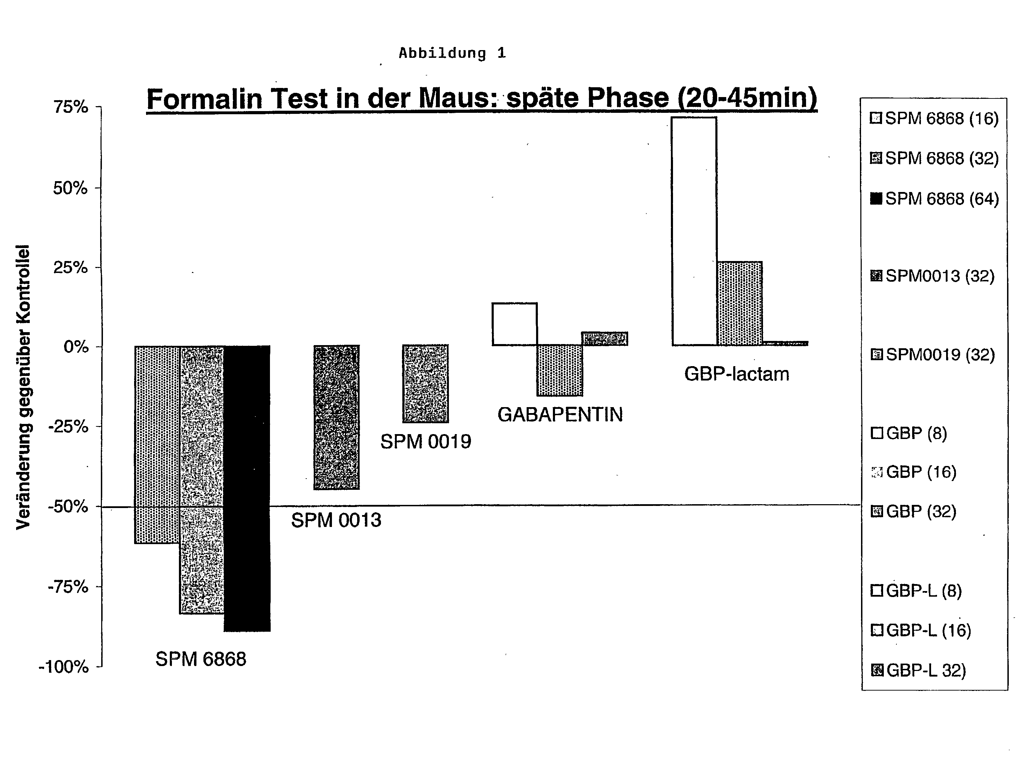

Abbildung 1 und Tabelle 1 zeigen jeweils die Reaktionen von Versuchstieren (je 10 Mäuse) 20 - 45 Minuten nach intraperitonealer Gabe ausgewählter Verbindungen. Dabei wurden jeweils als maximale Dosierung Konzentrationen der Azaspiroverbindungen gewählt, die um ca. einen Faktor 2 unterhalb der toxischen Dosis liegen, die zuvor im IRWIN-Test (Irwin, Psychopharmacologia 13 (1968) 222) bestimmt wurde.

Dabei haben die in Abbildung 1 verwendeten Abkürzungen die folgende Bedeutung: SPM 6868 entspricht 2-Azaspiro[4.6]undecan-3-thion, SPM 0013 entspricht 2-Azaspiro[4.5]decan-3-thion, SPM 0019 entspricht 2-Azaspiro[4.5]decan. GBP bedeutet Gabapentin. GBP-L bedeutet Gabapentin-Lactam. In der Legende wird jeweils in Klammern hinter der Substanzbezeichnung die Dosierung der Substanzen in mg/kg Körpergewicht angegeben.

Wie aus Abbildung 1 hervorgeht, haben die erfindungsgemäßen Verbindungen überraschenderweise im Formalintest eine signifikant höhere Potenz als Gabapentin und Gabapentin-Lactam, die als Vergleich eingesetzt wurden.

Darüberhinaus zeigten auch die beiden Verbindungen N-(2-Azaspiro[4.5]decan-3)-oxim (SPM 6850) und N-(2-Azaspiro[4.6]undecan-3)-oxim (SPM 6873) im oben beschriebenen Formalintest nach 30-45 Minuten eine mittlere Reduktion der Schmerzreaktion um 44% bzw. 36,5%, wie Tabelle 1 zeigt:

**Tabelle 1**

| Verbindung | Dosierung (mg/kg) | mittlere Abweichung der Schmerzreduktion gegenüber Kontrolle (%) (x Minute nach Formalingabe) | | |
|---|---|---|---|---|
| | | 20-25' | 30-35' | 40-45' |
| SPM 6868 | | | | |
| Meßreihe 1 (n=10) | 64 | - 89 | - 67 | - 83 |
| | 32 | - 83 | 0 | - 24 |
| | 16 | - 62 | - 29 | - 49 |
| Meßreihe 2 (n-10) | 64 | - 100 | - 98 | - 93 |
| | 16 | - 42 | -30 | -31 |
| | 4 | - 53 | - 7 | -16 |
| SPM 0013 (n=10) | 32 | - 99 | - 32 | - 4 |
| SPM 0019 (n=10) | 32 | - 67 | - 21 | - 26 |
| SPM 6850 (n=10) | 32 | (+) | -50 | - 38 |
| SPM 6873 (n=10) | 16 | (+) | - 35 | - 38 |

| GBP-L | | | | |
|---|---|---|---|---|
| Meßreihe 1 (n=10) | 32 | - 64 | - 15 | - 46 |
| | 16 | - 74 | - 40 | - 41 |
| | 8 | - 58 | - 18 | - 15 |
| Meßreihe 2 (n=10) | 32 | (+) | -10 | nb |
| | 16 | (+) | (+) | nb |
| | 8 | (+) | (+) | nb |
| Morphium* | 8 | - 87 | -95 | - 88 |

| | | | | |
|---|---|---|---|---|
| (+): Verstärkung der Schmerzreaktion *) Mittelwert aus 9 Meßreihen nb = nicht bestimmt | | | | |

Schließlich erwiesen sich die oberhalb der maximal tolerablen Dosen einsetzenden Nebenwirkungen der erfindungsgemäßen Verbindungen (Sedierung, Tremor, Hypothermie) als weit weniger schwer als diejenigen des GPL, bei dem eine signifikante Letalitätsrate zu beobachten war.

Die erfindungsgemäß für die Therapie geeigneten Azaspiroverbindungen sind somit besonders zur Behandlung von Schmerzen, insbesondere von chronischen, chronischentzündlichen und/oder neuropathischen Schmerzen geeignet.

Ein Gegenstand der Erfindung ist daher die Verwendung einer Azaspiroverbindung der Formeln
2-Azaspiro[4,5]decan-3-thion
2-Azaspiro[4,4]nonan-3-thion
2-Azaspiro[4,6]undecan-3-thion
N-(2-Azaspiro[4.5]decan-3)-oxim
N-(2-Azaspiro[4.6]undecan)-3-oxim
1-(2-Azaspiro[4.5]dec-2yl)-ethanon.
2-Azaspiro[4,5]decan
sowie möglicher Tautomere und/oder pharmazeutisch annehmbarer Salze
zur Herstellung eines Arzneimittels zur Behandlung von Schmerzen, insbesondere von chronischen, chronisch-entzündlichen und/oder neuropathischen Schmerzen.

Weiterhin können die Azaspiroverbindungen in Abhängigkeit von den Substituenten in verschiedenen tautomeren Formen vorliegen, die gegebenenfalls durch Salzbildung stabilisiert werden können.

Als pharmazeutisch annehmbare Salze kommen alle bioverträglichen Salze in Frage, die die pharmakologischen Eigenschaften der Wirkstoffe weitgehend erhalten und keine unerwünschten toxischen Effekte auslösen. Beispiele hierfür sind insbesondere Additionssalze anorganischer oder organischer Säuren, wie z.B. Hydrogenchlorid, Hydrogenbromid, Essigsäure, Zitronensäure, Weinsäure, Oxalsäure, Fumarsäure, Äpfelsäure, Bernsteinsäure oder Methansulfonsäure.

Die die erfindungsgemäßen Azaspiroverbindungen enthaltenden Arzneimittel können grundsätzlich zur Behandlung verschiedener Schmerzformen, wie z.B. Migräneschmerzen, Skelett- und Muskelschmerzen etc. verwendet werden. Besonders geeignet sind die die erfindungsgemäßen Azaspiroverbindungen enthaltenden Arzneimittel aber zur Behandlung von chronischen, chronisch-entzündlichen und/oder neuropathischen Schmerzen.

Neuropathischer Schmerz ist eine komplexe Erkrankung, die oft als Folgeerkrankung von Verletzungen, Infektionen, metabolischen Störungen und degenerativen Erkrankungen des Nervensystems auftreten kann. Beispiele für neuropathische Schmerzsyndrome sind beispielsweise Phantomschmerzen, postherpetische Neuralgien nach Gürtelrose, schmerzhafte diabetische Neuropathien, komplexe regionale Schmerzsyndrome, verschiedene Arten von Krebsschmerzen, neuropathische Schmerzen in Zusammenhang mit Multipler Sklerose oder mit Verletzungen größerer Nervenbahnen, dem Rückenmark oder dem Stammhirn.

Zur Herstellung eines Schmerzmittels besonders bevorzugt werden solche Azaspiroverbindungen, die im Formalintest, wie in Beispiel 8 beschrieben, in mindestens einem, bevorzugt in mindestens zwei der Testzeiträume (20-25', 30-35', 40-45' nach Formalingabe) eine mittlere Abweichung der Schmerzreaktion von wenigstens - 40 %, bevorzugt mindestens - 50 % oder - 60 % bewirken.

Unter dem Begriff "mittlere Abweichung der Schmerzreaktion" wird in dieser Anmeldung die relative Abweichung verstanden, die sich ergibt, wenn die gemittelte Zeit der Schmerzreaktion von 10 mit Wirkstoffen behandelten Tieren einer Versuchsreihe, wie in Ausführungsbeispiel 8 beschrieben, in einem definierten Zeitraum (20-25', 30-35' oder 40-45' nach Formalininjektion) mit der gemittelten Zeit der Schmerzreaktion von 10 mit Vehikel behandelten Kontrolltieren verglichen wird. Eine Reduktion der Schmerzreaktion wird dabei in negativen Prozentzahlen angegeben.

Die Erfindung wird durch die nachfolgenden Beispiele näher beschrieben.

### 1. Herstellung von 2-Azaspiro[4.5]decan-3-thion

In einem Einhalskolben wurden 1,01 g (6,6 mmol) Gabapentinlactam, 1,6 g (4,0 mmol) Lawessons' Reagenz und 20 ml Toluol vorgelegt. Die gelbe Lösung wurde unter Rückfluß (130°C) 20 Stunden gerührt. Nach dem Abkühlen wurde über eine Kieselgelsäule abfiltriert (Essigsäure als Laufmittel). Das Lösungsmittel wurde abgezogen.

Der gelbe Feststoff wurde noch einmal über eine Kieselgelsäule aufgereinigt und nachfolgend eine weitere Säulentrennung mit Aluminiumoxyd durchgeführt. Das Lösemittel wurde abgezogen und der Rückstand aus 40 ml Diisopropylether bei 4°C umkristallisiert.

Die Ausbeute betrug 25 % d.Th. (278 mg)

Der Schmelzpunkt beträgt 121,9°C (Büchi B-545, 1°C/min)

NMR (CDCl₃): 204.64; 60.19; 55.44; 42.18; 36.03; 25.35; 22.90.

### 2. Herstellung von 2-Azaspiro[4.4]nonan-3-thion

In einem Einhalskolben wurden 3,93 g (28,2 mmol) 2-Azaspiro[4.4]nonan-3-on, 6,5 g Lawessons Reagenz und 100 ml Toluol vorgelegt. Das Reaktionsgemisch wurde 20 Stunden unter Rückfluß erhitzt. Dabei entstand eine gelbe Lösung

Das Lösemittel wurde am Rotationsverdampfer abgezogen.

Der Rückstand wurde über eine Aluminiumoxidsäule chromatographiert (Laufmittel: Dichlormethan).

Das Laufmittel wurde abdestilliert und der zurückbleibende Feststoff in 100 ml Diisopropylether und 15 ml Dichlormethan mit Aktivkohle aufgekocht. Die Aktivkohle wurde heiß abfiltriert und das Filtrat auf +4°C gekühlt. Dabei fielen weiße Kristalle aus.
Die Ausbeute betrug 1,14 g (26 % d.Th.).

NMR (CDCl₃): 205.24; 60.50; 55.66; 49.42; 37.60; 23.73.

### 3. Herstellung von 2-Azaspiro[4.6]undecan-3-thion

In einem Einhalskolben wurden 4,18 g (25 mmol) 2-Azaspiro[4.6]undecan-3-on, 5,8 g (14,3 mmol) Lawessons' Reagenz und 125 ml Toluol vorgelegt und sodann unter Rückfluß für 18 Stunden refluxiert. Die abgekühlte Lösung wurde über Aluminiumoxid (neutral) abfiltiert. Das Filtrat wurde einrotiert und der Rückstand aus 60 ml Diisopropylether umkristallisiert.

Die Ausbeute betrug 1,2 g (26,2 % d.Th.).

Der Schmelzpunkt betrug 132,3°C (Büchi B-545, 1°C/min)

NMR: 205.05; 61.59; 57.32; 45.83; 39.50; 28.68; 23.26.

### 4. Herstellung von 2-Azaspiro[4.5]decan

In einem 100 ml-Dreihalskolben mit Magnetrührer, Tropftrichter und Rückflußkühler wurden 28 ml 1,0 M Lithiumaluminiumhydrid in THF (28,0 mmol) vorgelegt. Dazu wurde unter Rühren und Abkühlen auf 0°C-10°C eine Lösung von 5,01 g (32,7 mmol) Gabapentinlactam (2-Aza-spiro[4.5]decan-3-on) in 30 ml THF zugetropft. Diese Reaktion war sehr exotherm und fand unter H₂-Entwicklung statt.

Nach Beendigung des Zutropfens wurde noch 4 Stunden unter Rückfluß erhitzt.

Anschließend wurde das Reaktionsgemisch auf 0°C abgekühlt und mit einer Mischung von 2 ml Wasser und 2 ml THF versetzt, um das überschüssige LiAlH₄ zu vernichten. Nach der endgültigen Zersetzung wurden noch weitere 10 ml THF dazugegeben, um die Lösung zu verdünnen. Das Reaktionsgemisch wurde mit 10 g NaSO₄ versetzt und noch 10 Minuten gerührt.

Das Natriumsulfat wurde abfiltriert und dreimal mit jeweils 20 ml THF gewaschen. Das Lösemittel wurde abgezogen. Zurück blieb Amin als klares, farbloses Öl.

Die Ausbeute betrug 4,06 g (89,2 % d.Th.).

NMR (CDCl₃): 59.03; 46.28; 42.98; 38.72; 37.01; 25.66; 23.78.

### 5. Herstellung von N-(2-Azaspiro[4.5]decan-3)-oxim

1,69 g des wie in Beispiel 1 beschrieben hergestellten 2-Azaspiro[5.5.]decan-3-thion wurden in 200 ml Ethanol gelöst. Nach zugabe von 25 ml 50%iger Hydroxylaminlösung in Wasser wurde der Ansatz über Nacht bei Raumtemperatur gerührt.

Der Ansatz wurde vollständig einrotiert. Der ölige Rückstand wurde in 10 ml Wasser aufgenommen und kristallisierte nach kurzer Zeit durch. Das Produkt wurde abgesaugt und im Vakuum bei Raumtemperatur getrocknet.

Die Ausbeute des farblosen Produkts betrug 63,10 % d.Th. (1,06 g)

Der Schmelzpunkt wurde mit 156,9°C bestimmt (Büchi B-545, 1°C/min).

NMR (CDCl₃): 158.30; 55.59; 40.98; 38.86; 36.09; 25.93; 23.27.

### 6. Herstellung von N-(2-Azaspiro[4.6]undecan-3)-oxim

550 mg des wie unter Beispiel 3 beschrieben hergestellten 2-Azaspiro[4.6]undecan-3-thion wurden in 60 ml Ethanol gelöst. Nach Zugabe von 15 ml 50%ige Hydroxylaminlösung in Wasser wurde der Ansatz über Nacht bei Raumtemperatur gerührt.

Der Ansatz wurde vollständig einrotiert. Der ölige Rückstand wurde in 10 ml Wasser aufgenommen und kristallisierte nach kurzer Zeit durch. Das Produkt wurde abgesaugt und im Vakuum bei Raumtemperatur getrocknet.

Die Ausbeute des farblosen Produkts betrug 91,1 % d.Th (460 mg)

Der Schmelzpunkt wurde mit 140.8 °C bestimmt (Büchi B-545, 1°C/min).

NMR(CDCL₃):158.38; 56.90; 44.36; 40.87; 39.01; 29.21; 23.61; 23.25.

### 7. Herstellung von 1-(2-Azaspiro[4.5]dec-2yl)-ethanon

1,98 g des wie in Beispiel 4 beschrieben hergestellten 2-Azapiro[4.5.]decans wurden in 10 ml Dichlormethan verdünnt. Unter Argon wurden dazu 2.5 ml Triethylamin zugegeben und die Lösung auf 0°C abgekühlt. Unter Rühren wird eine Lösung von 1.2 ml Acetylchlorid in 10 ml Dichlormethan zugetropft. Danach wird 2 Stunden bei 0°C und eine Stunde bei Raumtemperatur gerührt.

Zum Reaktionsgemisch werden 20 ml Wasser gegeben und die Phasen getrennt. Die organische gelbe Phase wird mit 20 ml 1M HCl und mit 20 ml Wasser gewaschen. Die wässrigen Phasen werden mit Dichlormethan gewaschen. Die organischen Phasen werden über Natriumsulfat getrocknet, das Natriumsulfat abfiltriert und das lösungsmittel abdestilliert. Der Rückstand wird zweimal über eine Kieselgelsäule (Laufmittel: Triethylamin/Essigester 1:9) chromatographiert.

Die Ausbeute betrug 100% d.Th (2,54 g).

NMR (CDCl₃): 169.28; 58.03; 55.49; 45.74; 43.87; 35.17; 25.98; 23.20; 22.01.

### 8. In vivo-Test zur Bestimmung der analoetischen Wirksamkeit der Azaspiroverbindungen

Der Test wurde durchgeführt wie durch Wheeler-Aceto (Psychopharmacology, 104, 1991, 35) beschrieben.

NMRI-Mäuse mit einem Gewicht von 20-25 g wurden unter kontrollierten Bedingungen (22 ± 2°C, 40-70 % Luftfeuchtigkeit) gehalten. 25 µl einer 5 % Formcarbonyllösung wurde in die Hinterpfote injiziert und nachfolgend zu definierten Zeitpunkten (20, 30, 40 Minuten) für jeweils 5 Minuten die Zeit des Leckens der Pfoten gemessen.

Die höchstmögliche einsetzbare, nicht-toxische Konzentration der jeweiligen Testsubstanzen wurde zunächst im IRWIN-Test bestimmt (Irwin, Psychopharmacologia 13, 1968, 222).

Die Testsubstanzen wurden in physiologischer Kochsalzlösung mit 0,5 % Natriumcarboxymethylzellulose gelöst und jeweils in 3 Dosierungen gemessen, die 10 Minuten vor Formalingabe intraperitoneal appliziert wurden. Als Vergleichswert diente eine Vehikelkontrolle (10 ml/kg).

Der Test wurde verblindet mit je 10 Mäusen pro Versuchsreihe durchgeführt. Die Auswertung erfolgte durch einen Vergleich der behandelten Tiere mit Vehikelkontrollen zu drei verschiedenen Zeitpunkten. Hierzu wurde jeweils für die 10 Tiere einer Versuchsreihe pro Zeitraum ein Mittelwert für die Schmerzreaktion ermittelt und dann die relative Abweichung der mit Wirkstoff behandelten Tiere von den Kontrolltieren für jeden der drei verschiedenen Zeiträume bestimmt. Eine mittlere Reduktion der Schmerzreaktion für die behandelten Tiere um 50 % ergibt sich demzufolge, wenn für einen definierten Zeitraum (z.B. 30-35' nach Formalininjektion) die Zeit des Leckens der Pfote (gemittelt über die 10 Versuchstiere) gegenüber unbehandelten Tieren um 50 % reduziert ist. Die statistische Signifikanz wurde mittels Mann-Whitney U-Test ermittelt.

## Patentansprüche

1. Azaspiroverbindung ausgewählt aus der Gruppe
2-Azaspiro[4,5]decan-3-thion
2-Azaspiro[4,4]nonan-3-thion
2-Azaspiro[4,6]undecan-3-thion
N-(2-Azaspiro[4.5]decan-3)-oxim
N-(2-Azaspiro[4.6]undecan)-3-oxim
1-(2-Azaspiro[4.5]dec-2yl)-ethanon.

2. Azaspiroverbindung ausgewählt aus der Gruppe
2-Azaspiro[4,5]decan-3-thion
2-Azaspiro[4,4]nonan-3-thion
2-Azaspiro[4,6]undecan-3-thion
N-(2-Azaspiro[4.5]decan-3)-oxim
N-(2-Azaspiro[4.6]undecan)-3-oxim
1-(2-Azaspiro[4.5]dec-2yl)-ethanon.
2-Azaspiro[4,5]decan
zur Verwendung als Arzneimittel

3. Pharmazeutische Formulierung umfassend eine Verbindung nach Anspruch 2 und mindestens ein pharmazeutisches Hilfsmittel.

4. Verwendung einer Verbindung nach Anspruch 2 oder eines möglichen Tautomers und/oder pharmazeutisch annehmbaren Salzes zur Herstellung eines Arzneimittels zur Behandlung von Schmerzen.

5. Verwendung nach Anspruch 4, wobei der Schmerz chronischer, chronischentzündlicher und/oder neuropathischer Schmerz ist.

## Claims

1. Azaspiro compound selected out of the group encompassing
2-azaspiro[4.5]decane-3-thion
2-azaspiro[4.4]nonane-3-thion
2-azaspiro[4.6]undecane-3-thion
N-(2-azaspiro[4.5]decane-3)-oxim
N-(2-azaspiro[4.6]undecane)-3-oxim
1-(2-azaspiro[4.5]dec-2yl)-ethanon

2. Azaspiro compound selected out of the group encompassing
2-azaspiro[4.5]decane-3-thion
2-azaspiro[4.4]nonane-3-thion
2-azaspiro[4.6]undecane-3-thion
N-(2-azaspiro[4.5]decane-3)-oxim
N-(2-azaspiro[4.6]undecane)-3-oxim
1-(2-azaspiro[4.5]dec-2yl)-ethanon
2-azaspiro[4.5]decane
for use as medications

3. Pharmaceutical formulation encompassing a compound per claim 2 and at least one pharmaceutical adjuvant.

4. Use of a compound per claim 2 or of a possible tautomer and/or a pharmaceutically acceptable salt in producing a medication for the treatment of pain.

5. Use as in claim 4, where the pain is chronic, chronic-phlogistic and/or neuropathic pain.

## Revendications

1. Composé azaspiro choisi parmi le groupe composé de
2-azaspiro[4,5]décane-3-thione
2-azaspiro[4,4]nonane-3-thione
2-azaspiro[4,6]undécane-3-thione
N-(2-azaspiro[4,5]décane-3)-oxime
N-(2-azaspiro[4,6]undécane-3)-oxime
1-(2-azaspiro[4,5]déc-2-yle)-éthanone.

2. Composé azaspiro choisi parmi le groupe composé de
2-azaspiro[4,5]décane-3-thione
2-azaspiro[4,4]nonane-3-thione
2-azaspiro[4,6]undécane-3-thione
N-(2-azaspiro[4,5]décane-3)-oxime
N-(2-azaspiro[4,6]undécane-3)-oxime
1-(2-azaspiro[4,5]déc-2-yle)-éthanone
2-azaspiro[4,5]décane
et destiné à une utilisation comme médicament.

3. Formulation pharmaceutique comprenant un composé selon la revendication 2 et au moins un auxiliaire pharmaceutique.

4. Utilisation d'un composé selon la revendication 2 ou d'un tautomère possible et/ou d'un sel pharnaceutiquement acceptable en vue de la fabrication d'un médicament pour le traitement de douleurs.

5. Utilisation selon la revendication 4, la douleur étant une douleur chronique, inflammatoire chronique et/ou neuropathique.
